(19) Europäisches Patentamt
European Patent Office
Office européen des brevets

(11) Numéro de publication : **0 117 771**
**B1**

(12) # FASCICULE DE BREVET EUROPÉEN

(45) Date de publication du fascicule du brevet : **11.11.87**

(21) Numéro de dépôt : **84400081.0**

(22) Date de dépôt : **13.01.84**

(51) Int. Cl.⁴ : **C 07 D207/22**, C 07 D405/04, C 07 D405/12, A 61 K 31/40 // (C07D405/12, 317:64, 207:22),(C07D405/04, 307:81, 207:22)

(54) **Imino-2 pyrrolidines, leur procédé de préparation et leurs applications en thérapeutique.**

(30) Priorité : **28.01.83 FR 8301360**

(43) Date de publication de la demande : **05.09.84 Bulletin 84/36**

(45) Mention de la délivrance du brevet : **11.11.87 Bulletin 87/46**

(84) Etats contractants désignés : **AT BE CH DE FR GB IT LI LU NL SE**

(56) Documents cités :
**EP-A- 0 007 824**
**DE-A- 1 770 752**
**DE-A- 2 321 950**
**FR-A- 2 227 002**
**Le dossier contient des informations techniques présentées postérieurement au dépôt de la demande et ne figurant pas dans le présent fascicule.**

(73) Titulaire : **LABORATOIRES JACQUES LOGEAIS Société dite:**
**71, Avenue du Général de Gaulle**
**F-92130 Issy-Les-Moulineaux (FR)**

(72) Inventeur : **Maillard, Jacques**
**82 bis Avenue de Paris**
**F-78000 Versailles (FR)**
Inventeur : **Vo Van, Tri**
**5 bis rue du Centre**
**F-91430 Igny (FR)**
Inventeur : **Legeai, Jacky**
**3 Boulevard Diderot**
**F-91120 Palaiseau (FR)**
Inventeur : **Benharkate, Marguerite**
**7 Résidence des Trois Ormes**
**F-78610 Le Perray en Yvelines (FR)**

(74) Mandataire : **Polus, Camille et al**
**c/o Cabinet Lavoix 2, Place d'Estienne d'Orves**
**F-75441 Paris Cedex 09 (FR)**

EP 0 117 771 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des imino-2 pyrrolidines qui peuvent être utilisées en thérapeutique dans le domaine cardiovasculaire.

DE-A-2 321 950 décrit des dérivés d'amino-2 phényl-4 méthyl-5 1-pyrroline comme ayant une action sur le système nerveux central et sur le système cardiovasculaire.

EP-A-0 007 824 décrit des dérivés d'amino-2 (benzofuryl-2)-4 Δ1-pyrroline comme ayant une action dans le domaine cardiovasculaire, notamment une action antidysrythmique.

La présente invention a pour objet des composés de formule

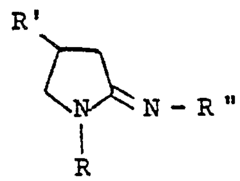

(I)

dans laquelle

R représente un groupe alcoyle en $C_1$-$C_4$, un groupe alcényle en $C_2$-$C_4$ ou un groupe alcynyle en $C_2$-$C_4$

R' représente un groupe phényle éventuellement substitué par un ou plusieurs substituants choisis parmi un atome de chlore, un atome de fluor, un groupe alcoyle en $C_1$-$C_4$, un groupe alcoxy en $C_1$-$C_4$, un groupe hydroxy, un groupe nitrile ou un groupe méthylène dioxy ; un groupe benzofuryle-2 ou un groupe phénoxy méthyle éventuellement substitué sur la partie phényle par un ou plusieurs substituants choisis parmi un atome de chlore, un atome de fluor, un groupe alcoyle en $C_1$-$C_4$, un groupe hydroxy, un groupe nitrile ou un groupe méthylène dioxy,

R" représente un groupe alcoyle en $C_1$-$C_4$, un groupe alcényle en $C_2$-$C_4$, un groupe alcynyle en $C_2$-$C_4$, un groupe hydroxy alcoyle en $C_1$-$C_4$, un groupe (alcoxy en $C_1$-$C_4$) (alcoyle en $C_1$-$C_4$), un groupe phényl (alcoyle en $C_1$-$C_4$) ou phénoxy (alcoyle en $C_1$-$C_4$) éventuellement substitués sur la partie phényle par un ou plusieurs substituants choisis parmi un atome de chlore, un atome de fluor, un groupe alcoyle en $C_1$-$C_4$, un groupe alcoxy en $C_1$-$C_4$, un groupe hydroxy, un groupe nitrile ou un groupe méthylène dioxy,

ainsi que leurs sels d'addition avec des acides pharmaceutiquement acceptables.

La présente invention a également pour objet des compositions thérapeutiques contenant à titre de principe actif un composé de formule I ou l'un de ses sels d'addition avec des acides pharmaceutiquement acceptables.

Dans la définition ci-dessus on désigne par « sels d'addition avec des acides pharmaceutiquement acceptables » les sels qui possèdent les propriétés biologiques des bases libres, sans avoir d'effet indésirable. Ces sels peuvent être ceux formés avec des acides minéraux tels que l'acide chlorhydrique, l'acide bromhydrique, l'acide sulfurique, l'acide nitrique, l'acide phosphorique, des sels métalliques acides tels que l'orthophosphate disodique et le sulfate monopotassique, et des acides organiques tels que l'acide formique, l'acide acétique, l'acide propionique, l'acide glycolique, l'acide oxalique, l'acide fumarique, l'acide citrique, l'acide malique, l'acide méthane sulfonique, l'acide lactique, l'acide succinique, l'acide tartrique et l'acide pamoïque.

Une classe préférée de composés de formule I est celle dans laquelle R" représente un groupe phényl (alcoyle en $C_1$-$C_4$) ou phénoxy (alcoyle en $C_1$-$C_4$) substitué ou non, et plus particulièrement un groupe phényl (alcoyle en $C_1$-$C_4$), diméthoxy-3,4 phényl (alcoyle en $C_1$-$C_4$) et phénoxy (alcoyle en $C_1$-$C_4$).

Pour préparer les composés de formule I, on peut opérer comme suit :

a) on effectue une alcoylation d'une pyrrolidinone-2 de formule

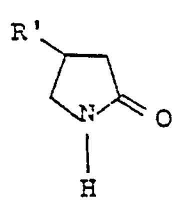

(II)

dans laquelle R' a la signification donnée ci-dessus, de façon à obtenir une pyrrolidinone-2 de formule

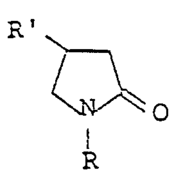

(III)

b) on convertit la pyrrolidinone-2 de formule III en une alcoxy-2 Δ2-pyrroline de formule

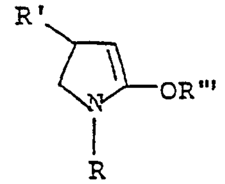

(IV)

dans laquelle R et R′ ont les significations données précédemment et R‴ représente un groupe méthyle ou éthyle,

c) on fait réagir sur une alcoxy-2 Δ2-pyrroline de formule IV une amine de formule R″—$NH_2$, obtenant ainsi un composé de formule I, et éventuellement on convertit le composé de formule I ainsi obtenu en un sel avec un acide pharmaceutiquement acceptable.

Les pyrrolidinones-2 de formule II sont connues (Brevet FR-A-2 100 946) ou peuvent être préparées comme les composés connus.

L'alcoylation des pyrrolidinones-2 de formule II peut être effectuée de manière classique. C'est ainsi que l'on peut faire réagir sur une pyrrolidinone-2 de formule II un agent d'alcoylation tel qu'un halogénure, sulfate ou tosylate. La réaction suivant les cas peut être effectuée par simple chauffage ou après transformation de la pyrrolidinone en pyrrolidinone sodée au moyen d'hydrure de sodium. En variante, on peut effectuer la réaction en présence d'une base quaternaire, selon la méthode classique de transfert de phase.

Dans le cas où le radical R′ représente un groupe phénoxyméthyle non substitué ou substitué, on effectue avantageusement l'alcoylation d'une pyrrolidinone-2 de formule

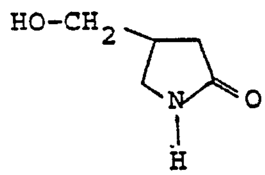

et l'on convertit ensuite le groupe OH en un groupe phénoxy, notamment par passage par le tosylate et réaction avec un phénolate alcalin.

La conversion de la pyrrolidinone-2 de formule III en alcoxy-2 Δ2-pyrroline de formule IV peut être effectuée par chauffage avec du sulfate de diméthyle, puis avec de l'éthylate ou du méthylate de sodium.

La conversion peut également être effectuée par action de tétrafluoroborate de triéthyloxonium (L. F. Fieser et M. Fieser Reagents for Organic Synthesis Wiley, vol. 1, p. 1210) à température ordinaire, de préférence dans un solvant chloré. Dans ce cas, l'alcoxy-2 Δ2-pyrroline n'est généralement pas isolée, mais traitée directement dans le stade suivant.

La réaction des alcoxy-2 Δ2-pyrrolines de formule IV avec les amines primaires R″ $NH_2$ peut généralement être effectuée par simple mélange à température ordinaire.

Les exemples suivants illustrent la préparation des composés de formule I.

## Exemple 1

Méthyl-1 phényl-4 phénéthylimino-2 pyrrolidine et hydrogénofumarate (I,R = $—CH_3$, R′ = $—C_6H_5$, R″ = $—CH_2CH_2C_6H_5$)

a) Méthyl-1 phényl-4 pyrrolidinone-2

A une solution de 16,1 g (0,1 mole) de phényl-4 pyrrolidinone-2 dans 40 ml de benzène portée à 60-70 °C, on ajoute 12,6 g (0,1 mole) de sulfate de diméthyle. Le mélange est porté à reflux pendant 3 h. Après alcalinisation, la phase organique est décantée et la phase aqueuse est extraite au benzène. Les phases benzéniques réunies sont séchées sur pastilles de soude et évaporées. Le résidu est distillé sous pression réduite : $E_{10}$ = 180 °C.

Variante par transfert de phase.

On agite pendant 5 h à 36 °C un mélange de 32,2 g (0,2 mole) de phényl-4 pyrrolidinone-2, 37,8 g (0,3 mole) de sulfate de diméthyle, 3,4 g (0,01 mole) d'hydrogénosulfate de tétrabutylammonium, 100 ml de solution aqueuse de soude à 50 % et 200 ml de toluène. Après refroidissement, la phase aqueuse est décantée, extraite au chlorure de méthylène qu'on joint à la phase toluénique. La phase organique est lavée à l'eau, séchée et évaporée. Le résidu est distillé : $E_{0.2}$ = 120 °C. Rendt. 79 %.

b) Méthyl-1 phényl-4 éthoxy-2 Δ2-pyrroline

Un mélange de 23 g (0,13 mole) du produit obtenu en (a) et de 16,6 g (0,13 mole) de sulfate de diméthyle est agité pendant 3 h à 80 °C. Après baisse de la température à 50-60 °C, on ajoute le mélange à une solution d'éthylate de sodium préparée à partir de 2,9 g de sodium dans 55 ml d'éthanol absolu.

On agite 6 h à 50-60 °C, puis évapore l'éthanol. Le résidu est distillé sous pression réduite : $E_{0,1}$ = 104-114 °C.

c) Méthyl-1 phényl-4 phénéthylimino-2 pyrrolidine et hydrogénofumarate

On porte à reflux pendant 24 h une solution contenant 11 g (0,54 mole) du composé obtenu en (b) et 5 ml de phénéthylamine dans 70 ml d'éthanol. Après évaporation du solvant, le résidu est distillé : $E_{0,1}$ = 166 °C. Rendt. 67 %.

La base obtenue est transformée en sel par addition d'une quantité équimoléculaire d'acide fumarique dans l'éthanol à reflux. L'alcool est évaporé et le résidu cristallisé par addition d'éther. F = 124 °C. Rendt. quantitatif.

Exemple 2

Méthyl-1 phényl-4 phénylisopropylimino-2 pyrrolidine et hydrogénofumarate

$$(I, R = -CH_3,\ R' = -C_6H_5,\ R'' = -\underset{CH_3}{\underset{|}{CH}}-CH_2C_6H_5)$$

La base est préparée comme dans l'exemple 1 à partir de méthyl-1 phényl-4 éthoxy-2 Δ2-pyrroline et de phénylisopropylamine. $E_{0,1}$ = 150-152 °C. Rendt. 61 %.

La base est transformée en hydrogénofumarate comme décrit dans l'exemple 1. F = 110 °C. Rendt. quantitatif.

Exemple 3

Méthyl-1 phényl-4 (phénoxy-2 éthylimino)-2 pyrrolidine et hydrogénofumarate. (I,R = $-CH_3$, R' = $-C_6H_5$, R" = $-CH_2CH_2OC_6H_5$)

La base est préparée comme dans l'exemple 1 à partir de méthyl-1 phényl-4 éthoxy-2 Δ2-pyrroline et de phénoxy-2 éthylamine. $E_{0,2}$ = 188-190 °C. Rendt. 70 %.

La base est transformée en hydrogénofumarate comme décrit dans l'exemple 1. F : 104 °C. Rendt. quantitatif.

Exemple 4

Méthyl-1 phényl-4 (diméthoxy-3',4'-phénéthylimino)-2 pyrrolidine et hydrogénofumarate

(I,R = $-CH_3$, R' = $-C_6H_5$, R" = $-CH_2CH_2-$ 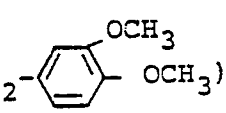 )

La base est préparée comme dans l'exemple 1, à partir de méthyl-1 phényl-4 éthoxy-2 Δ2-pyrroline et de diméthoxy-3',4' phénéthylamine. $E_{0,2}$ = 216 °C. Rendt. 59 %.

La base est transformée en hydrogénofumarate, comme décrit dans l'exemple 1. Le sel cristallise dans un mélange d'isopropanol et d'éther diisopropylique. F = 216 °C. Rendt. 75 %.

Exemple 5

Ethyl-1 phényl-4 (diméthoxy-3',4'-phénéthylimino)-2 pyrrolidine et phosphate

(I,R = $-C_2H_5$, R' = $-C_6H_5$, R" = $-CH_2-CH_2-$ 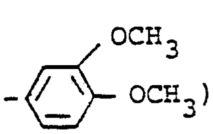 )

a) Ethyl-1 phényl-4 pyrrolidinone-2

Un mélange de 24,2 g (0,15 mole) de phényl-4 pyrrolidinone-2 et 5,4 g (0,18 mole) d'hydrure de

sodium en suspension huileuse à 80 %, dans 150 ml de tétrahydrofuranne est porté à reflux pendant 2 h 30 mn. Après refroidissement, on ajoute 29 g (0,185 mole) d'iodure d'éthyle et porte à nouveau à reflux pendant 5 h. Le solvant est évaporé et le résidu est repris par le chloroforme, lavé à l'eau, séché et distillé. $E_{0,2}$ = 124-126 °C. Rendt. 57 %.

b et c) Ethyl-1 phényl-4 (diméthoxy-3',4'-phénéthylimino)-2 pyrrolidine et phosphate

Un mélange de 7,55 g (0,04 mole) d'éthyl-1 phényl-4 pyrrolidinone-2, 8,6 g (0,04 mole) de tétrafluoroborate de triéthyloxonium et 100 ml de chlorure de méthylène est abandonné pendant 4 jours à température ordinaire, près refroidissement dans un bain d'eau et de glace, on ajoute 9,1 g (0,05 mole) de diméthoxy-3',4' phénéthylamine, agite pendant 3 h et laisse reposer pendant une nuit. Le mélange est alors hydrolysé par addition de 40 ml de soude aqueuse 2,5 N. La phase organique est décantée, lavée à l'eau, séchée sur carbonate de potassium et distillée. $E_{0,3}$ = 198 °C. Rendt. 56 %.

La base est transformée en phosphate monobasique par addition d'une quantité équimoléculaire d'acide phosphorique dans l'éthanol. Le sel cristallise après agitation pendant 2 h. F = 128 °C. Rendt. 98 %.

Exemple 6

n.Propyl-1 phényl-4 (phénoxyéthyl-imino)-2 pyrrolidine et hydrogénofumarate. (I,R = n.$C_3H_7$, R' = $-C_6H_5$, R" = $-CH_2CH_2O-C_6H_5$)

a) n.Propyl-1 phényl-4 pyrrolidinone-2

Elle est préparée par action du bromure de n.propyle sur la phényl-4 pyrrolidinone-2, en présence d'hydrure de sodium, comme décrit dans l'exemple 5. $E_{0,3}$ = 128-130 °C. Rendt. 62 %.

b et c) n.Propyl-1 phényl-4 (phénoxyéthyl-imino) 2 pyrrolidine et hydrogénofumarate.

La base est préparée à partir de propyl-1 phényl-4 pyrrolidinone-2 par actions successives du tétrafluoborate de triéthyloxonium, puis de la phénoxyéthylamine, comme décrit dans l'exemple 5. $E_{0,2}$ = 186-187 °C. Rendt. 98 %.

La base est transformée en hydrogénofumarate, comme décrit dans l'exemple 1. F = 126 °C. Rendt. 98 %.

Exemple 7

Allyl-1 phényl-4 (diméthoxy-3',4'-phénéthyl-imino)-2 pyrrolidine et phosphate

(I,R= $-CH_2CH=CH_2$, R' = $-C_6H_5$, R" = $-CH_2CH_2-$ 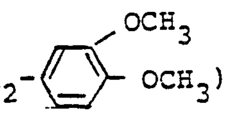)

a) Allyl-1 phényl-4 pyrrolidinone-2

Elle est préparée par action du bromure d'allyle sur la phényl-4 pyrrolidinone-2, en présence d'hydrure de sodium, comme décrit dans l'exemple 5. $E_{0,3}$ = 128 °C. Rendt. 75 %.

b et c) Allyl-1 phényl-4 (diméthoxy-3',4'-phénéthyl-imino)-2 pyrrolidine et phosphate

La base est préparée par actions successives du tétrafluoroborate de triéthyloxonium et de la diméthoxy-3',4'-phénéthylamine sur l'allyl-1 phényl-4 pyrrolidinone-2 comme décrit dans l'exemple 5. $E_{0,3}$ = 222-224 °C. Rendt. 70 %.

La base est transformée en phosphate monobasique comme dans l'exemple 5. F = 106 °C.

Exemple 8

Méthyl-1 (benzofuryl-2)-4 n.propyl-imino-2 pyrrolidine et hydrogénofumarate

(I,R = $-CH_3$ , R' = 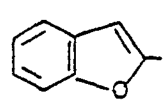 , R" = $-nC_3H_7$ )

a) Méthyl-1 (benzofuryl-2)-4 pyrrolidinone-2

Elle est préparée à partir de (benzofuryl-2)-4 pyrrolidinone-2, d'hydrure de sodium et d'iodure de méthyle, comme décrit dans l'exemple 5. $E_{0,3}$ = 172 °C. F = 73 °C. Rendt. 81 %.

b et c) Méthyl-1 (benzofuryl-2)-4 n.propyl-imino-2 pyrrolidine et hydrogénofumarate

La base est préparée à partir de méthyl-1 (benzofuryl-2)-4 pyrrolidinone-2 par actions successives de tétrafluoroborate de triéthyloxonium et de n.propyl-amine comme décrit dans l'exemple 5. $E_{0,2}$ = 150-152 °C. Rendt. 78 %.

La base est transformée en hydrogénofumarate comme dans l'exemple 1. Rendt. 86 %.

Exemple 9

Méthyl-1 (benzofuryl-2)-4 allylimino-2 pyrrolidine et hydrogénofumarate

(I,R = $-CH_3$ , R' = 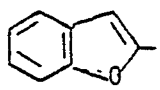 , R" = $-CH_2CH{=}CH_2$)

La base est préparée à partir de méthyl-1 (benzofuryl-2)-4 pyrrolidinone-2 par actions successives du tétrafluoroborate de triéthyloxonium et d'allylamine, comme décrit dans l'exemple 5. $E_{0,1}$ = 156-158 °C. Rendt. 85 %.

La base est transformée en hydrogénofumarate comme dans l'exemple 1. F = 136 °C. Rendt. 89 %.

Exemple 10

Méthyl-1 (benzofuryl-2)-4 propargylimino-2 pyrrolidine et hydrogénofumarate

(I,R = $-CH_3$, R' = 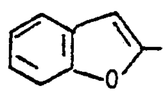 , R" = $-CH_2C{\equiv}CH$)

La base est préparée à partir de méthyl-1 (benzofuryl-2)-4 pyrrolidinone-2 par actions successives du tétrafluoroborate de triéthyloxonium et de propargylamine, comme décrit dans l'exemple 5. $E_{0,3}$ = 168-170 °C. Rendt. 72 %.

La base est transformée en hydrogénofumarate, comme dans l'exemple 1. F = 152 °C. Rendt. 76 %.

Exemple 11

Méthyl-1 (benzofuryl-2)-4 (hydroxy-2 éthylamino)-2 pyrrolidine et hydrogénofumarate

(I,R = $-CH_3$ , R' = 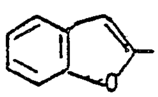 , R" = $-CH_2CH_2OH$)

La base est préparée à partir de méthyl-1 (benzofuryl-2)-4 pyrrolidinone-2 par actions successives de tétrafluoborate de triéthyloxonium et d'hydroxy-2 éthylamine, comme décrit dans l'exemple 5. $E_{0,5}$ = 184 °C. Rendt. 52 %.

La base est transformée en hydrogénofumarate recristallisé dans l'isopropanol. F = 121 °C. Rendt. 56 %.

Exemple 12

Méthyl-1 (benzofuryl-2)-4 (méthoxy-2 éthylimino)-2 pyrrolidine et hydrogénofumarate

(I,R = $-CH_3$ , R' = 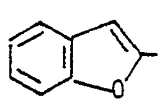 , R" = $-CH_2CH_2OCH_3$ )

La base est préparée à partir de méthyl-1 (benzofuryl-2)-4 pyrrolidinone-2 par actions successives de tétrafluoroborate de triéthyloxonium et de méthoxy-2 éthylamine, comme dans l'exemple 5. $E_{0,1}$ = 168-170 °C. Rendt. 78 %.

La base est transformée en hydrogénofumarate comme dans l'exemple 1. F = 136 °C. Rendt. 87 %.

Exemple 13

Méthyl-1 (benzofuryl-2)-4 phénéthylimino-2 pyrrolidine et méthanesulfonate

$(I, R = -CH_3$ , R' = 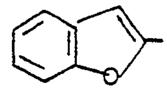 , $R'' = -CH_2CH_2C_6H_5)$

La base est préparée à partir de méthyl-1 (benzofuryl-2)-4 pyrrolidinone-2 par actions successives de tétrafluoroborate de triéthyloxonium et de phénéthylamine comme dans l'exemple 5. $E_{0,1}$ = 200-202 °C.

La base est transformée en méthane sulfonate par addition d'un léger défaut d'acide au sein de l'acétate d'éthyle où le sel cristallise. Rendt. 86 %.

## Exemple 14

Méthyl-1 (benzofuryl-2)-4 phénylisopropyl-imino-2 pyrrolidine et méthane sulfonate

$(I, R = -CH_3$ , R' = 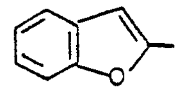 , $R'' = -\underset{\displaystyle CH_3}{\underset{|}{C}}H-CH_2C_6H_5)$

La base est préparée à partir de méthyl-1 (benzofuryl-2)-4 pyrrolidinone-2 par actions successives du tétrafluoroborate de triéthyloxonium et de phénylisopropylamine comme dans l'exemple 5. $E_{0,1}$ = 180-185 °C. Rendt. 66 %.

La base est transformée en méthane sulfonate comme décrit dans l'exemple 13. F = 172 °C.

## Exemple 15

Méthyl-1 (benzofuryl-2)-4 (phénoxy-2 éthyl-imino)-2 pyrrolidine et hydrogénofumarate

$(I, R = -CH_3$ , R' = 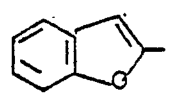 , $R'' = -CH_2CH_2O-C_6H_5)$

La base est préparée à partir de méthyl-1 (benzofuryl-2)-4 pyrrolidinone-2 par actions successives du tétrafluoroborate de triéthyloxonium et de phénoxy-2 éthylamine comme dans l'exemple 5. $E_{0,2}$ = 212-218 °C. Rendt. 73 %.

La base est transformée en hydrogénofumarate, comme dans l'exemple 1. F = 129 °C. Rendt. 90 %.

## Exemple 16

Méthyl-1 (benzofuryl-2)-4 (diméthoxy-3',4'-phénéthylimino)-2 pyrrolidine et hydrogénofumarate

$(I, R = -CH_3$ , R' = 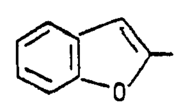 , $R'' = -CH_2CH_2-$ 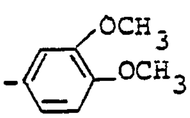 )

La base est préparée à partir de méthyl-1 (benzofuryl-2)-4 pyrrolidinone-2 par actions successives de tétrafluoroborate de triéthyloxonium et de (diméthoxy-3',4'-phényl)-2 éthylamine, comme dans l'exemple 5. $E_{0,1}$ = 226 °C. Rendt. 93 %.

La base est transformée en hydrogénofumarate comme dans l'exemple 1. F = 106 °C. Rendt. 93 %.

## Exemple 17

Méthyl-1 phénoxyméthyl-4 (phénoxy-2 éthylimino)-2 pyrrolidine et hydrogénofumarate. $(I, R = -CH_3$, $R' = -CH_2-O-C_6H_5$, $R'' = -CH_2CH_2O-C_6H_5)$

a) Méthyl-1 phénoxyméthyl-4 pyrrolidinone-2

On prépare d'abord la méthyl-1 tosyloxyméthyl-4 pyrrolidinone-2 par action de 110 g (0,58 mole) de chlorure de p.toluènesulfonyle sur 68 g (0,53 mole) de méthyl-1 hydroxyméthyl-4 pyrrolidinone-2 dans 170 ml de pyridine, en refroidissant à 10 °C. Après 10 h à température ambiante le mélange est hydrolysé sur de la glace et extrait au chlorure de méthylène. L'évaporation du solvant laisse un résidu cristallin. F = 66 °C. Rendt. 76 %.

Une solution de 21,5 g (0,075 mole) du tosylate ainsi obtenu et 10 g (0,075 mole) de phénate de potassium dans 150 ml de diméthylformamide est portée à reflux pendant 2 h.

Après concentration à sec sous vide, le résidu est repris par du chlorure de méthylène, lavé à l'eau, séché et distillé. $E_{0,3}$ = 154-156 °C. Rendt. 79 %.

b et c) Méthyl-1 phénoxyméthyl-4 (phénoxy-2 éthylimino)-2 pyrrolidine et hydrogénofumarate

La base est préparée à partir de la pyrrolidinone-2 obtenue au stade (a) par actions successives du tétrafluoroborate de triéthyloxonium et de la phénoxy-2 éthylamine, comme décrit dans l'exemple 5. $E_{0,3}$ = 210 °C. Rendt. 58 %.

La base est transformée en hydrogénofumarate, comme décrit dans l'exemple 1. F = 144 °C. Rendt. 94 %.

## Exemple 18

Méthyl-1 phénoxyméthyl-4 (diméthoxy-3',4'-phénéthylimino)-2 pyrrolidine et phosphate

(I,R = $-CH_3$ , R' = $-CH_2-O-C_6H_5$ , R" = $-CH_2CH_2-$ 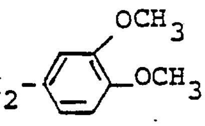 )

La base est préparée à partir de méthyl-1 phénoxyméthyl-4 pyrrolidinone-2 par actions successives de tétrafluoroborate de triéthyloxonium et de la diméthoxy-3',4'-phénéthylamine, comme décrit dans l'exemple 5. $E_{0,3}$ = 230 °C. Rendt. 62 %.

La base est transformée en phosphate monobasique comme décrit dans l'exemple 5. F = 230° (dec) ; Rendt. 62 %.

## Exemple 19

Méthyl-1 (méthylène dioxy-3',4'-phénoxyméthyl)-4 (phénoxy-2 éthylimino)-2 pyrrolidine et hydrogénofumarate

(I,R = $-CH_3$ , R' = 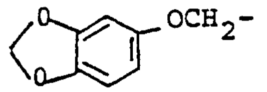 , R" = $-CH_2CH_2O-C_6H_5$)

a) Méthyl-1 (méthylène dioxy-3',4'-phénoxyméthyl)-4 pyrrolidinone-2

Elle est préparée à partir de la méthyl-1 tosyloxyméthyl-4 pyrrolidinone-2 décrite dans l'exemple 17 et du méthylène dioxy-3,4-phénate de potassium, comme décrit dans l'exemple 17. $E_{0,2}$ = 190 °C. Rendt. 69 %.

b et c) Méthyl-1 (méthylène dioxy-3',4'-phénoxyméthyl)-4 (phénoxy-2 éthylimino)-2 pyrrolidine et hydrogénofumarate

La base est préparée à partir de la pyrrolidinone obtenue au stade (a) par actions successives du tétrafluoroborate de triéthyloxonium et de la phénoxy-2 éthylamine, comme décrit dans l'exemple 5. $E_{0,2}$ = 200 °C. Rendt. 73 %.

La base est transformée en hydrogénofumarate comme décrit dans l'exemple 1. F = 148 °C. Rendt. 78 %.

## Exemple 20

Méthyl-1 (méthylènedioxy-3',4'-phénoxyméthyl)-4 (diméthoxy-3',4'-phénéthylimino)-4 pyrrolidine et phosphate

(I,R = $-CH_3$ , R' = 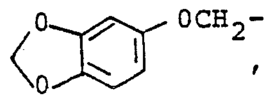 , R" = $-CH_2CH_2-$ 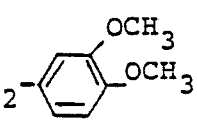 )

La base est préparée à partir de la pyrrolidinone décrite dans l'exemple 19, par actions successives du tétrafluoroborate de triéthyloxonium et de la diméthoxy-3',4'-phénéthylamine, comme décrit dans l'exemple 5. $E_{0,3}$ = 256 °C. Rendt. 66 %.

La base est transformée en phosphate monobasique, comme dans l'exemple 5. F = 211 °C. Rendt. 80 %.

## Exemple 21

Méthyl-1 (éthyl-2'-phénoxyméthyl)-4 (diméthoxy-3',4'-phénéthylimino)-2 pyrrolidine et phosphate

(I,R = $-CH_2$ , R' = 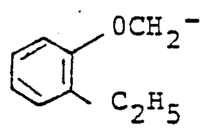 , R" = $-CH_2CH_2-$ 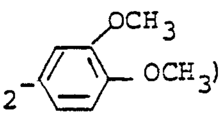)

a) Méthyl-1 (éthyl-2'-phénoxyméthyl)-4 pyrrolidinone-2

Elle est préparée à partir de la méthyl-1 tosyloxyméthyl-4 pyrrolidinone-2, décrite dans l'exemple 17, et de l'éthyl-2 phénate de potassium, comme décrit dans l'exemple 17. $E_{0,3}$ = 186 °C.

b et c) Méthyl-1 (éthyl-2'-phénoxyméthyl)-4 (diméthoxy-3',4'-phénéthylimino)-2 pyrrolidine et phosphate

La base est préparée à partir de la pyrrolidinone obtenue au stade (a) par actions successives du tétrafluoroborate de triéthyloxonium et de la diméthoxy-3',4'-phénéthylamine, comme dans l'exemple 5. $E_{0,2}$ = 224 °C. Rendt. 49 %.

La base est transformée en phosphate monobasique comme dans l'exemple 5. F = 208 °C. Rendt. 91 %.

## Exemple 22

Méthyl-1 (cyano-2' phénoxyméthyl)-4 (phénoxy-2 éthylimino)-2 pyrrolidine et hydrogénofumarate

(I,R = $-CH_3$, R' = 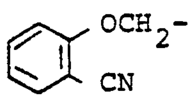 , R" = $-CH_2CH_2O-C_6H_5$)

a) Méthyl-1 (cyano-2'-phénoxyméthyl)-4 pyrrolidinone-2

Elle est préparée à partir de la tosyloxyméthyl-4 pyrrolidinone-2 décrite dans l'exemple 17 et du cyano-2 phénate de sodium, comme décrit dans l'exemple 17. $E_{0,2}$ = 196 °C. Rendt. 68 %.

b et c) Méthyl-1 (cyano-2' phénoxyméthyl)-4 (phénoxy-2 éthylimino)-2 pyrrolidine et hydrogénofumarate

La base est préparée à partir de la pyrrolidinone obtenue en (a) par actions successives du tétrafluoroborate de triéthyloxonium et de phénoxy-2 éthylamine, comme dans l'exemple 5.

La base est transformée en hydrogénofumarate comme décrit dans l'exemple 1. F = 160 °C. Rendt. global : 52 %.

## Exemple 23

Méthyl-1 (méthoxy-3 phényl)-4 (phénoxy-2 éthylimino)-2 pyrrolidine et hydrogénofumarate

(I,R = $-CH_3$. R' = 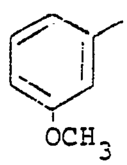 , R" = $-CH_2CH_2O-C_6H_5$)

a) (Méthoxy-3 phényl)-4 pyrrolidinone-2

Elle est préparée comme les pyrrolidinones-2 de formule II décrites dans FR-A-2 100 946.

b) Méthyl-1 (méthoxy-3 phényl)-4 pyrrolidinone-2

Elle est préparée par méthylation de la pyrrolidinone-2 précédente, selon le procédé décrit dans l'exemple 1.

$E_{0,4}$ = 150 °C — Rendt : 73 %

c) Méthyl-1 (méthoxy-3 phényl)-4 (phénoxy-2 éthylimino)-2 pyrrolidine et hydrogénofumarate

La base est préparée à partir de la pyrrolidinone obtenue en (b) par actions successives du tétrafluoborate de triéthyloxonium et de phénoxy-2 éthylamine, comme décrit dans l'exemple 5.

$E_{0,3}$ = 209 °C — Rendt : 46 %

La base est transformée en hydrogénofumarate comme décrit dans l'exemple 1.

F = 142 °C — Rendt : 89 %

Exemple 24

Méthyl-1 (chloro-2 phényl)-4 (phénoxy-2 éthylimino)-2 pyrrolidine et hydrogénofumarate

(I, R = $CH_3$ , R' = 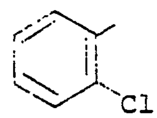 R" = $-CH_2CH_2OC_6H_5$)

a) (Chloro-2 phényl)-4 pyrrolidinone-2

Elle est préparée comme les pyrrolidinones-2 de formule II décrites dans FR-A-2 100 946.

b) Méthyl-1 (chloro-2 phényl)-4 pyrrolidinone-2

Elle est préparée par méthylation de la pyrrolidinone-2 précédente selon le procédé décrit dans l'exemple 1.
$E_{0,3}$ = 132 °C — Rendt : 74 %.

c) Méthyl-1 (chloro-2 phényl)-4 (phénoxy-2 éthylimino)-2 pyrrolidine et hydrogénofumarate

La base est préparée à partir de la pyrrolidinone obtenue en (b), par actions successives du tétrafluoroborate de triéthyloxonium et de phénoxy-2 éthylamine comme décrit dans l'exemple 5.
$E_{0,1}$ = 192 °C — Rendt : 42 %.
La base est transformée en hydrogénofumarate comme décrit dans l'exemple 1.
F = 170 °C — Rendt : 92 %.

Exemple 25

Méthyl-1 (fluoro-4 phényl)-4 (phénoxy-2 éthylimino)-2 pyrrolidine et hydrogénofumarate

(I, R = $CH_3$ , R' = 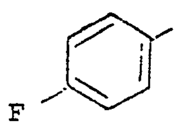 , R" = $-CH_2CH_2OC_6H_5$)

a) (fluoro-4 phényl)-4 pyrrolidinone-2

Elle est préparée comme les pyrrolidinones-2 de formule II décrites dans FR-A-2 100 946.

b) Méthyl-1 (fluoro-4 phényl)-4 pyrrolidinone-2

Elle est préparée par méthylation de la pyrrolidinone-2 précédente, selon le procédé décrit dans l'exemple 1.
$E_{0,3}$ = 128 °C — Rendt : 83 %.

c) Méthyl-1 (fluoro-4 phényl)-4 (phénoxy-2 éthylimino)-2 pyrrolidine et hydrogénofumarate

La base est préparée à partir de la pyrrolidinone obtenue en (b) par actions successives du tétrafluoroborate de triéthyloxonium et de phénoxy-2 éthylamine, comme décrit dans l'exemple 5.
$E_{0,1}$ = 184 °C — Rendt : 22 %
La base est transformée en hydrogénofumarate comme dans l'exemple 1.
F = 107 °C — Rendt : 83 %.

Les composés de formule I et leurs sels d'addition avec des acides pharmaceutiquement acceptables possèdent des propriétés intéressantes sur le cœur et les vaisseaux.

Ils ont notamment une activité antiagrégante. De plus, pour les composés préférés de formule I, c'est-à-dire ceux dans lesquels R" est un groupe phényl alcoyle ou phénoxyalcoyle, on observe une activité anti-arythmique intéressante.

Par ailleurs, la toxicité de ces composés n'apparaît qu'à des doses très supérieures aux doses pharmacologiquement actives, ce qui permet leur utilisation en thérapeutique, notamment en pathologie cardiovasculaire.

On donnera ci-après des résultats des études toxicologiques et pharmacologiques mettant en évidence ces propriétés.

Toxicité aiguë chez la souris

Les composés ont été administrés par voie orale ou intrapéritonéale, en solution dans le soluté physiologique (NaCl 0,9 %), à des souris mâles de souche Swiss dont le poids était compris entre 22 et 25 g. La mortalité a été notée 8 jours après traitement. Les doses administrées ont été de 10, 30, 100 et 200 mg/kg.

Les résultats sont donnés dans le Tableau I.

Tableau I

| Sels des exemples n° | Toxicité aiguë dose | voie | mortalité | Sels des exemples n° | Toxicité aiguë dose | voie | mortalité |
|---|---|---|---|---|---|---|---|
| 1 | 100<br>200 | i.p.<br>p.o. | 80 %<br>0 % | 14 | 100<br>200 | i.p.<br>p.o. | 100 %<br>0 % |
| 2 | 100<br>200 | i.p.<br>p.o. | 100 %<br>83 % | 15 | 100<br>200 | i.p.<br>p.o. | 100 %<br>0 % |
| 3 | 200<br>200 | i.p.<br>p.o. | 0 %<br>0 % | 16 | 100<br>200 | i.p.<br>p.o. | 100 %<br>0 % |
| 4 | 200<br>200 | i.p.<br>p.o. | 100 %<br>0 % | 18 | 100<br>200 | i.p.<br>p.o. | 40 %<br>0 % |
| 5 | 100<br>200 | i.p.<br>p.o. | 20 %<br>0 % | 19 | 100<br>200 | i.p.<br>p.o. | 80 %<br>0 % |
| 9 | 100<br>100 | i.p.<br>p.o. | 60 %<br>0 % | 20 | 100<br>200 | i.p.<br>p.o. | 80 %<br>0 % |
| 11 | 200<br>200 | i.p.<br>p.o. | 80 %<br>0 % | 21 | 33<br>200 | i.p.<br>p.o. | 20 %<br>0 % |
| 12 | 100<br>200 | i.p.<br>p.o. | 100 %<br>0 % | 22 | 33<br>200 | i.p.<br>p.o. | 40 %<br>0 % |
| 13 | 100<br>200 | i.p.<br>p.o. | 100 %<br>0 % | A* | 200<br>200 | i.p.<br>p.o. | 60 %<br>0 % |

A* Composé de comparaison : méthyl-1 [(diméthoxy-3,4 phénéthyl) imino]-pyrrolidine

Activité antiagrégante

Agrégation plaquettaire au collagène.

Des échantillons sanguins ont été prélevés chez le lapin, à l'artère carotide, sur anticoagulant (citrate de sodium à 3,8 %, 1 volume pour 9 volumes de sang). Le plasma riche en plaquettes (PRP) a été obtenu par centrifugation lente (1 200 t/mn pendant 10 mn), puis le plasma pauvre en plaquettes par centrifugation rapide 4 500 t/mn pendant 15 mn). Les échantillons de PRP (0,36 ml) ont été placés dans la cuve d'un agrégomètre, incubés à 37 °C, additionnés de tampon de Michaelis renfermant le composé étudié (0,04 ml), puis enfin de l'agent agrégant (collagène : 0,08 ml de suspension de collagène préalablement incubée à 33 °C pendant 90 secondes). L'agrégation plaquettaire se traduit par la diminution de la densité optique du PRP (D. O. initiale du PRP : 100 %, D. O. initiale du PPP = 0 %).

Les résultats sont donnés dans le Tableau II.

(Voir Tableau II page 12)

Tableau II

| Sel de l'exemple n° | Agrégation plaquettaire au collagène Concentrations (µg/ml) liminaires (inhibition de l'agrégation <50 %) | Concentrations (µg/ml) fortement inhibitrices (inhibition de l'agrégation >50 %) |
|---|---|---|
| 1 | | 100 |
| 2 | 10 | 30 - 100 |
| 3 | 1 - 3 | 10 - 100 |
| 4 | 10 - 30 | 100 |
| 5 | 3 - 10 | 30 - 100 |
| 6 | 1 - 3 | 10 - 100 |
| 7 | 10 | 30 - 100 |
| 8 | 10 - 30 | 100 |
| 9 | 3 - 10 | 30 - 100 |
| 10 | 100 | |
| 11 | 10 - 100 | |
| 12 | 3 - 30 | 100 |
| 13 | 3 - 30 | 100 |
| 14 | 3 - 10 | 30 - 100 |
| 16 | 10 - 30 | 100 |
| 17 | 3 | 10 - 100 |
| 18 | 10 - 30 | 100 |
| 19 | 3 - 10 | 30 - 100 |
| 20 | 10 - 30 | 100 |
| 21 | 3 | 10 - 100 |
| 22 | 30 | 100 |
| A | inactif | |

Pour un certain nombre de composés, on a en outre mis en évidence une activité fibrinolytique. La méthode suivante a été utilisée.

Des caillots de plasma humain ont été préparés par recalcification (une goutte de thrombase calcique à 20 U/ml pour 0,5 ml de plasma), de manière que le caillot se forme autour du crochet d'une tige de verre. Les caillots sont ensuite suspendus dans des solutions tamponnées (tampon véronal pH 7,2-7,4) maintenus à 37 °C. La lyse éventuelle du caillot est notée après 24 h et 48 h d'incubation.

Les résultats sont donnés dans le tableau III.

(Voir Tableau III page 13)

Tableau III

| Sel de l'exemple | Concentration (mmoles/ml) entraînant une lyse du caillot en 24 h | en 48 h |
|---|---|---|
| 1 | | 10 - 20 |
| 2 | 20 | |
| 3 | 10 - 20 | |
| 6 | | 5 - 10 |
| 7 | | 10 - 20 |
| 8 | | 20 |
| 13 | 10 | 2,5 - 5 |
| 18 | | 20 |
| 21 | | 10 - 20 |
| 22 | 20 | 5 - 10 |

Activité antidysrythmique

Test à l'aconitine chez le rat.

Les animaux étant anesthésiés à l'uréthane (1 g/kg i. p.), une injection de sulfate d'aconitine (27,5 µg/kg) était pratiquée dans la veine jugulaire préalablement cathétérisée, 30 minutes après l'administration par la même voie de l'un des composés. Le délai d'apparition de la première salve de dysrythmies était observé sur l'électrocardiogramme enregistré en dérivation D2 et comparé avec des animaux témoins.

Les résultats sont donnés dans le Tableau IV

Tableau IV

| Sel de l'exemple | Dysrythmies à l'aconitine dose mg/kg | % d'animaux protégés | Sel de l'exemple | Dysrythmies à l'aconitine dose mg/kg | % d'animaux protégés |
|---|---|---|---|---|---|
| 1 | 1 | 25 % | 16 | 3 | 80 % |
| 2 | 1 | 67 % | 17 | 10 | 100 % |
| 3 | 10 | 83 % | 18 | 5 | 100 % |
| 4 | 7,5 | 90 % | 19 | 2,5 | 25 % |
| 13 | 5 | 50 % | 20 | 10 | 100 % |
| 14 | 5 | 100 % | 21 | 7,5 | 100 % |
| 15 | 5 | 80 % | A | 5 | 0 % |

Ces résultats mettent en particulier en évidence le grand intérêt que présente le composé de l'exemple 3. Ce composé, qui est le moins toxique chez la souris, possède une activité antiagrégante, une activité antiarythmique, une activité fibrinolytique, ainsi qu'une activité α-adrénolytique (observée chez le chien après injection de nor-adrénaline).

Il trouve un intérêt particulier dans le traitement des problèmes cardiovasculaires consécutifs à l'infarctus du myocarde par exemple, ou la prévention des troubles du rythme cardiaque chez les patients à risque reconnu.

Les composés de formule I et leurs sels d'addition avec des acides pharmaceutiquement acceptables peuvent être administrés à l'homme sous forme de compositions pharmaceutiques par voie parentérale, orale, rectale ou percutanée.

Pour la voie parentérale, les compositions thérapeutiques peuvent être constituées par des sels en solutions aqueuses contenant éventuellement un adjuvant de solubilisation tel que l'alcool benzylique ou le propylène glycol, ou au sein d'un excipient assurant une résorption retardée.

Pour les autres voies, les compositions peuvent être constituées notamment par des comprimés, gélules, microgranules, suppositoires, pommades ou crèmes pouvant comporter à titre d'adjuvants les excipients habituels de ces formes pharmaceutiques.

Pour la voie orale, les compositions peuvent, en outre être constituées par des solutions aqueuses de sels hydrosolubles contenant éventuellement un adjuvant de solubilisation.

Les différentes compositions peuvent contenir de 5 à 500 mg de principe actif par unité de prise, selon les formes et les voies d'administration. La posologie journalière peut varier de 0,15 à 3 mg/kg selon la voie d'administration et les indications thérapeutiques.

**Revendications** (pour les Etats contractants : BE, CH, DE, FR, GB, IT, LI, LU, NL, SE)

1. Composés de formule

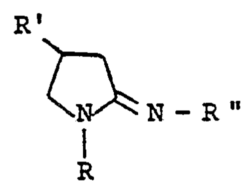

(I)

dans laquelle

R représente un groupe alcoyle en $C_1$-$C_4$, un groupe alcényle en $C_2$-$C_4$ ou un groupe alcynyle en $C_2$-$C_4$

R' représente un groupe phényle éventuellement substitué par un ou plusieurs substituants choisis parmi un atome de chlore, un atome de fluor, un groupe alcoyle en $C_1$-$C_4$, un groupe alcoxy en $C_1$-$C_4$, un groupe hydroxy, un groupe cyano ou un groupe méthylène dioxy ; un groupe benzofuryle-2 ou un groupe phénoxy méthyle éventuellement substitué sur la partie phényle par un ou plusieurs substituants choisis parmi un atome de chlore, un atome de fluor, un groupe alcoyle en $C_1$-$C_4$, un groupe hydroxy, un groupe cyano ou un groupe méthylène dioxy,

R" représente un groupe alcoyle en $C_1$-$C_4$, un groupe alcényle en $C_2$-$C_4$, un groupe alcynyle en $C_2$-$C_4$, un groupe hydroxy alcoyle en $C_1$-$C_4$, un groupe (alcoxy en $C_1$-$C_4$) (alcoyle en $C_1$-$C_4$), un groupe phényl (alcoyle en $C_1$-$C_4$) ou phénoxy (alcoyle en $C_1$-$C_4$) éventuellement substitués sur la partie phényle par un ou plusieurs substituants choisis parmi un atome de chlore, un atome de fluor, un groupe alcoyle en $C_1$-$C_4$, un groupe alcoxy en $C_1$-$C_4$, un groupe hydroxy, un groupe cyano ou un groupe méthylène dioxy,

ainsi que leurs sels d'addition avec des acides pharmaceutiquement acceptables.

2. Composés selon la revendication 1, caractérisés en ce que R" représente un groupe phényle (alcoyle en $C_1$-$C_4$) ou phénoxy (alcoyle en $C_1$-$C_4$) substitué ou non.

3. Composés selon la revendication 2, caractérisés en ce que R" représente un groupe phényl (alcoyle en $C_1$-$C_4$) diméthoxy-3,4 phényl (alcoyle en $C_1$-$C_4$) ou phénoxy (alcoyle en $C_1$-$C_4$).

4. Méthyl-1-phényl-4 (phénoxy-2 éthylimino)-2 pyrrolidine et ses sels d'addition avec des acides pharmaceutiquement acceptables.

5. Méthyl-1 phényl-4 (diméthoxy-3',4'-phénéthylimino)-2 pyrrolidine et ses sels d'addition avec des acides pharmaceutiquement acceptables.

6. Composition thérapeutique, caractérisée en ce qu'elle contient à titre de principe actif un composé selon l'une quelconque des revendications 1 à 5.

7. Composition selon la revendication 6, sous forme de dose unitaire, chaque dose contenant de 5 à 500 mg de principe actif.

8. Procédé de préparation d'un composé selon la revendication 1, caractérisé en ce que

a) on effectue une alcoylation d'une pyrrolidinone-2 de formule

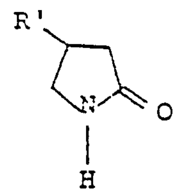

(II)

dans laquelle R' a la signification donnée ci-dessus, de façon à obtenir une pyrrolidinone-2 de formule

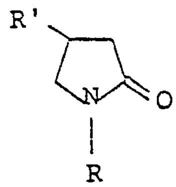

(III)

b) on convertit la pyrrolidinone-2 de formule III en une alcoxy-2 Δ2-pyrroline de formule

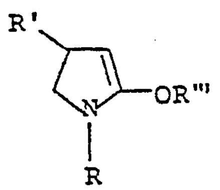

(IV)

dans laquelle R et R' ont les significations données précédemment et R''' représente un groupe méthyle ou éthyle,

c) on fait réagir sur une alcoxy-2 Δ2-pyrroline de formule IV une amine de formule R"—$NH_2$, obtenant ainsi un composé de formule I, et éventuellement on convertit le composé de formule I ainsi obtenu en un sel avec un acide pharmaceutiquement acceptable.

**Revendications** (pour l'Etat contractant AT)

1. Procédé de préparation de composés de formule

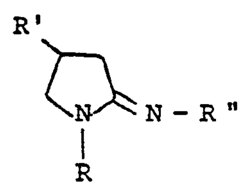

(I)

dans laquelle

R représente un groupe alcoyle en $C_1$-$C_4$, un groupe alcényle en $C_2$-$C_4$ ou un groupe alcynyle en $C_2$-$C_4$

R' représente un groupe phényle éventuellement substitué par un ou plusieurs substituants choisis parmi un atome de chlore, un atome de fluor, un groupe alcoyle en $C_1$-$C_4$, un groupe alcoxy en $C_1$-$C_4$, un groupe hydroxy, un groupe cyano ou un groupe méthylène dioxy ; un groupe benzofuryle-2 ou un groupe phénoxy méthyle éventuellement substitué sur la partie phényle par un ou plusieurs substituants choisis parmi un atome de chlore, un atome de fluor, un groupe alcoyle en $C_1$-$C_4$, un groupe hydroxy, un groupe cyano ou un groupe méthylène dioxy,

R" représente un groupe alcoyle en $C_1$-$C_4$, un groupe alcényle en $C_2$-$C_4$, un groupe alcynyle en $C_2$-$C_4$, un groupe hydroxy alcoyle en $C_1$-$C_4$, un groupe (alcoxy en $C_1$-$C_4$) (alcoyle en $C_1$-$C_4$), un groupe phényl (alcoyle en $C_1$-$C_4$) ou phénoxy (alcoyle en $C_1$-$C_4$) éventuellement substitués sur la partie phényle par un ou plusieurs substituants choisis parmi un atome de chlore, un atome de fluor, un groupe alcoyle en $C_1$-$C_4$, un groupe alcoxy en $C_1$-$C_4$, un groupe hydroxy, un groupe cyano ou un groupe méthylène dioxy,

ainsi que de leurs sels d'addition avec des acides pharmaceutiquement acceptables, caractérisé en ce que

a) on effectue une alcoylation d'une pyrrolidinone-2 de formule

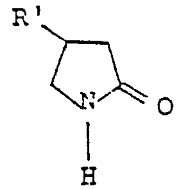

(II)

dans laquelle R′ a la signification donnée ci-dessus, de façon à obtenir une pyrrolidinone-2 de formule

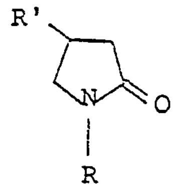

(III)

b) on convertit la pyrrolidinone-2 de formule III en une alcoxy-2 Δ2-pyrroline de formule

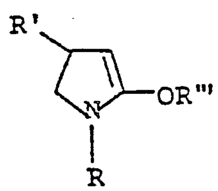

(IV)

dans laquelle R et R′ ont les significations données précédemment et R‴ représente un groupe méthyle ou éthyle,

c) on fait réagir sur une alcoxy-2 Δ2-pyrroline de formule IV une amine de formule R″—$NH_2$, obtenant ainsi un composé de formule I, et éventuellement on convertit le composé de formule I ainsi obtenu en un sel avec un acide pharmaceutiquement acceptable.

2. Procédé de préparation d'une composition thérapeutique, caractérisé en ce que l'on met un composé de formule I, tel que défini à la revendication 1, ou l'un de ses sels d'addition avec un acide pharmaceutiquement acceptable sous une forme thérapeutiquement administrable.

**Claims** (for the Contracting States : BE, CH, LI, DE, FR, GB, IT, NL, LU, SE)

1. Compounds of the formula

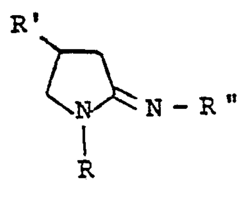

(I)

in which

R represents a $C_{1-4}$ alkyl group, a $C_{2-4}$ alkenyl group or a $C_{2-4}$ alkynyl group,

R′ represents a phenyl group optionally substituted with one or more substituents selected from a chlorine atom, a fluorine atom, a $C_{1-4}$ alkyl group, a $C_{1-4}$ alkoxy group, a hydroxy group, a cyano group or a methylene dioxy group ; a 2-benzofuryl group or a phenoxy methyl group optionally substituted on the phenyl moiety with one or more substituents selected from a chlorine atom, a fluorine atom, a $C_{1-4}$ alkyl group, a hydroxy group, a cyano group or a methylene dioxy group,

R″ represents a $C_{1-4}$ alkyl group, a $C_{2-4}$ alkenyl group, a $C_{2-4}$ alkynyl group, a $C_{1-4}$ hydroxyalkyl group, a ($C_{1-4}$ alkoxy) ($C_{1-4}$ alkyl) group, a phenyl ($C_{1-4}$ alkyl) or phenoxy ($C_{1-4}$ alkyl) group optionally substituted on the phenyl moiety with one or more substituents selected from a chlorine atom, a fluorine atom, a $C_{1-4}$ alkyl group, a $C_{1-4}$ alkoxy group, a hydroxy group, a cyano group, or a methylene dioxy group,

and their pharmaceutically acceptable acid addition salts.

2. Compounds as claimed in claim 1, wherein R″ represents a substituted or unsubstituted phenyl

($C_{1-4}$ alkyl) or phenoxy ($C_{1-4}$ alkyl) group.

3. Compounds as claimed in claim 2, wherein R″ represents a phenyl ($C_{1-4}$ alkyl), 3,4-dimethoxyphenyl ($C_{1-4}$ alkyl) or phenoxy ($C_{1-4}$ alkyl) group.

4. 1-Methyl-4-phenyl-2-(2-phenoxy-ethylimino) pyrrolidine and its pharmaceutically acceptable acid addition salts.

5. 1-Methyl-4-phenyl-2-(3′,4′-dimethoxy-phenethyl-imino) pyrrolidine and its pharmaceutically acceptable acid addition salts.

6. Therapeutic composition, characterized in that it comprises as active ingredient a compound as claimed in any one of claims 1-5.

7. Composition as claimed in claim 6, in unit dosage form, each unit dose containing 5-500 mg active ingredient.

8. Process for the preparation of a compound as claimed in claim 1, characterized by :

a) alkylating a pyrrolidin-2-one of the formula

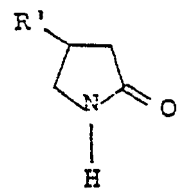

(II)

in which R′ is as defined in claim 1, to give a pyrrolidin-2-one of the formula :

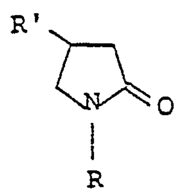

(III)

b) converting the pyrrolidin-2-one of the formula (III) to a 2-alkoxy-2-pyrroline of the formula

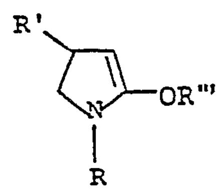

(IV)

in which R and R′ are as defined in claim 1 and R‴ represents a methyl or ethyl group.

c) reacting a 2-alkoxy-Δ2-pyrroline of the formula (IV) with an amine of the formula R″—$NH_2$, to give a compound of the formula (I), and optionally converting the resulting compound of the formula (I) to a pharmaceutically acceptable acid addition salt.

**Claims** (for the Contracting State AT)

1. Process for the preparation of compounds of the formula :

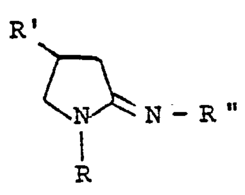

(I)

in which :

R represents a $C_{1-4}$ alkyl group, a $C_{2-4}$ alkenyl group or a $C_{2-4}$ alkynyl group,

R′ represents a phenyl group optionally substituted with on or more substituents selected from a chlorine atom, a fluorine atom, a $C_{1-4}$ alkyl group, a $C_{1-4}$ alkoxy group ; a hydroxy group, a cyano group, or a methylene dioxy group ; a 2-benzofuryl group or a phenoxy methyl group optionally substituted on

the phenyl moiety with one or more substituents selected from a chlorine atom, a fluirine atom, a $C_{1-4}$ alkyl group, a hydroxy group, a cyano group or a methylene dioxy group,

R″ represents a $C_{1-4}$ alkyl group, a $C_{2-4}$ alkenyl group, a $C_{2-4}$ alkynyl group, a $C_{1-4}$ hydroxyalkyl group, a ($C_{1-4}$ alkoxy) ($C_{1-4}$ alkyl) group, a phenyl ($C_{1-4}$ alkyl) or phenoxy ($C_{1-4}$ alkyl) group optionally substituted on the phenyl moiety with one or more substituents selected from a chlorine atom, a fluorine atom, a $C_{1-4}$ alkyl group, a $C_{1-4}$ alkoxy group, a hydroxy group, a cyano group, or a methylene dioxy group,

and of their pharmaceutically acceptable acid addition salts, characterized by :

a) alkylating a pyrrolidin-2-one of the formula

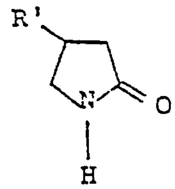

in which R′ is as defined in claim 1, to give a pyrrolidin-2-one of the formula :

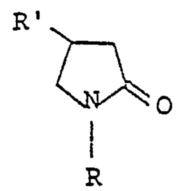

(III)

b) converting the pyrrolidin-2-one of the formula (III) to a 2-alkoxy-2-pyrrolidine of the formula

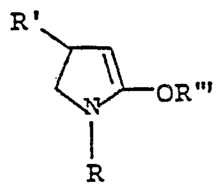

(IV)

in which R and R′ are as defined in claim 1 and R″ represents a methyl or ethyl group,

c) reacting a 2-alkoxy-Δ 2-pyrroline of the formula (IV) with an amine of the formula R″—$NH_2$, to give a compound of the formula (I), and optionally converting the resulting compound of the formula (I) to a pharmaceutically acceptable acid addition salt.

2. Process for the preparation of a therapeutic composition characterized in that a coumpound of formula I, as defined in claim 1 or one of its pharmaceutically acceptable acid addition salts is put under a therapeutically administrable form.

**Patentansprüche** (für die Vertragsstaaten : BE, CH, DE, FR, GB, IT, LI, LU, NL, SE)

1. Verbindungen der Formel

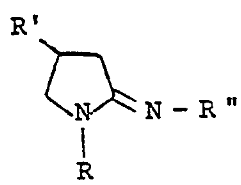

(I)

in der

R eine $C_1$-$C_4$-Alkylgruppe, eine $C_2$-$C_4$-Alkenylgruppe oder eine $C_2$-$C_4$-Alkinylgruppe darstellt,

R′ eine Phenylgruppe, die gegebenenfalls durch einen oder mehrere Substituenten, ausgewählt aus einem chloratom, einem Fluoratom, einer $C_1$-$C_4$-Alkylgruppe, einer $C_1$-$C_4$-Alkoxygruppe, einer Hydroxygruppe, einer Cyanogruppe oder einer Methylendioxygruppe, substituiert ist ; eine 2-Benzofurylgruppe oder eine Phenoxymethylgruppe, die gegebenenfalls an ihrem Phenylteil durch eine oder mehrere

Substituenten, ausgewählt aus einem Chloratom, einem Fluoratom, einer $C_1$-$C_4$-Alkylgruppe, einer Hydroxygruppe, einer Cyanogruppe oder einer Methylendioxygruppe substituiert ist, bedeutet,

R″ eine $C_1$-$C_4$-Alkylgruppe, eine $C_2$-$C_4$-Alkenylgruppe, eine $C_2$-$C_4$-Alkinylgruppe, eine Hydroxy-$C_1$-$C_4$-alkylgruppe, eine $C_1$-$C_4$-Alkoxy-$C_1$-$C_4$-alkylgruppe, eine Phenyl-$C_1$-$C_4$-alkylgruppe oder eine Phenoxy-$C_1$-$C_4$-alkylgruppe, die gegebenenfalls an ihrem Phenylteil mit einem oder mehreren Substituenten ausgewählt aus einem Chloratom, einem Fluoratom, einer $C_1$-$C_4$-Alkylgruppe, einer $C_1$-$C_4$-Alkoxygruppe, einer Hydroxygruppe, einer Cyanogruppe oder einer Methylendioxygruppe substituiert ist, bedeutet, sowie wie ihre Additionssalze mit pharmazeutisch unbedenklichen Säuren.

2. Verbindung gemäß Anspruch 1, dadurch gekennzeichnet, daß R″ eine Phenyl-$C_1$-$C_4$-alkylgruppe oder eine Phenoxy-$C_1$-$C_4$-alkylgruppe, die substituiert oder unsubstituiert ist, darstellt.

3. Verbindung gemäß Anspruch 2, dadurch gekennzeichnet, daß R″ eine Phenyl-$C_1$-$C_4$-alkylgruppe, eine 3,4-Dimethoxyphenyl-$C_1$-$C_4$-alkylgruppe oder eine Phenoxy-$C_1$-$C_4$-alkylgruppe darstellt.

4. 1-Methyl-4-phenyl(2-phenoxyethylimino)-2-pyrrolidin und seine Additionssalze mit pharmazeutisch unbedenklichen Säuren.

5. 1-Methyl-4-phenyl(3′,4′-dimethoxy-phenylethylimino)-2-pyrrolidin und seine Additionssalze mit pharmazeutisch unbedenklichen Säuren.

6. Therapeutische Zubereitung, dadurch gekennzeichnet, daß sie als Wirkstoff eine Verbindung gemäß irgendeinem der Ansprüche 1-5 enthält.

7. Zubereitung gemäß Anspruch 6 in Form einer Einzeldosis, wobei jede Dosis 5-500 mg des Wirkstoffes enthält.

8. Verfahren zur Herstellung von Verbindungen gemäß Anspruch 1, dadurch gekennzeichnet, daß man

a) eine Alkylierung eines 2-Pyrrolidinons der Formel

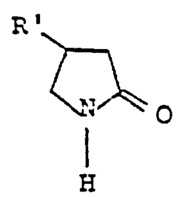

(II)

in der R′ die oben angegebene Bedeutung hat, derart bewirkt, daß man ein 2-Pyrrolidinon der Formel (III) erhält,

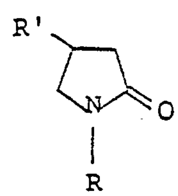

(III)

b) man das 2-Pyrrolidinon der Formel (III) in ein 2-Alkoxy-Δ2-pyrrolin der Formel (IV) umwandelt,

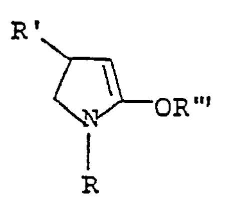

(IV)

in der R und R′ die vorstehend genannten Bedeutungen haben und R‴ eine Methyl- oder Ethylgruppe darstellt,

c) das 2-Alkoxy-Δ2-pyrrolin der Formel (IV) mit einem Amin der Formel R″—$NH_2$ reagieren läßt, und so eine Verbindung der Formel (I) erhält, und gegebenenfalls die so erhaltene Verbindung der Formel (I) in ein Salz mit einer pharmazeutisch unbedenklichen Säure umwandelt.

**Patentansprüche** (für den Vertragsstaat AT)

1. Verfahren zur Herstellung von Verbindungen der Formel

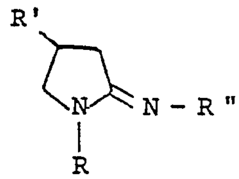 (I)

in der

R eine $C_1$-$C_4$-Alkylgruppe, eine $C_2$-$C_4$-Alkenylgruppe oder eine $C_2$-$C_4$-Alkinylgruppe darstellt,

R′ eine Phenylgruppe, die gegebenenfalls durch einen oder mehrere Substituenten, ausgewählt aus einem Chloratom, einem Fluoratom, einer $C_1$-$C_4$-Alkylgruppe, einer $C_1$-$C_4$-Alkoxygruppe, einer Hydroxygruppe, einer Cyanogruppe oder einer Methylendioxygruppe, substituiert ist ; eine 2-Benzofurylgruppe oder eine Phenoxymethylgruppe, die gegebenenfalls an ihrem Phenylteil durch eine oder mehrere Substituenten, ausgewählt aus einem Chloratom, einem Fluoratom, einer $C_1$-$C_4$-Alkylgruppe, einer Hydroxygruppe, einer Cyanogruppe oder einer Methylendioxygruppe substituiert ist, bedeutet,

R″ eine $C_1$-$C_4$-Alkylgruppe, eine $C_2$-$C_4$-Alkenylgruppe, eine $C_2$-$C_4$-Alkinylgruppe, eine Hydroxy-$C_1$-$C_4$-alkylgruppe, eine $C_1$-$C_4$-Alkoxy-$C_1$-$C_4$-alkylgruppe, eine Phenyl-$C_1$-$C_4$-alkylgruppe oder eine Phenoxy-$C_1$-$C_4$-alkylgruppe, die gegebenenfalls an ihrem Phenylteil mit einem oder mehreren Substituenten, ausgewählt aus einem Chloratom, einem Fluoratom, einer $C_1$-$C_4$-Alkylgruppe, einer $C_1$-$C_4$-Alkoxygruppe, einer Hydroxygruppe, einer Cyanogruppe oder einer Methylendioxygruppe substituiert ist, bedeutet, sowie ihre Additionssalze mit pharmazeutisch unbedenklichen Säuren, dadurch gekennzeichnet, daß man

a) eine Alkylierung eines 2-Pyrrolidinons der Formel

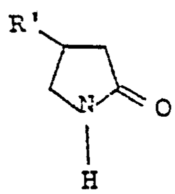 (II)

in der R′ die oben angegebene Bedeutung hat, derart bewirkt, daß man ein 2-Pyrrolidinon der Formel (III) erhält,

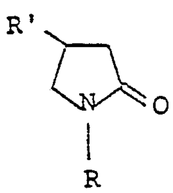 (III)

b) man das 2-Pyrrolidinon der Formel (III) in ein 2-Alkoxy-Δ2-pyrrolin der Formel (IV) umwandelt,

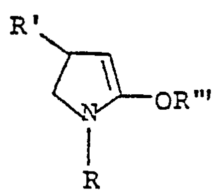 (IV)

in der R und R′ die vorstehend genannten Bedeutungen haben und R‴ eine Methyl- oder Ethylgruppe darstellt,

c) das 2-Alkoxy-Δ2-pyrrolin der Formel (IV) mit einem Amin der Formel R″—$NH_2$ reagieren läßt, und so eine Verbindung der Formel (I) erhält und gegebenenfalls die so erhaltene Verbindung der Formel (I) in ein Salz mit einer pharmazeutisch unbedenklichen Säure umwandelt.

2. Verfahren zur Herstellung einer therapeutischen Zubereitung, dadurch gekennzeichnet, daß man eine Verbindung der Formel (I) verwendet, wie sie in Anspruch 1 definiert ist, oder eines ihrer Additionssalze mit pharmazeutisch unbedenklichen Säuren in einer therapeutisch anwendbaren Form.